# EUROPEAN PATENT APPLICATION

(11) **EP 3 330 694 A1**
(43) Date of publication of application: **06.06.2018**
(21) Application number: 16830152.1
(22) Date of filing: 31.05.2016
(51) Int. Cl.: G01N 1/30, G01N 1/28, G01N 33/48

(54) **SPECIMEN STAINING SYSTEM**

(30) Priority: 29.07.2015 JP 2015149590
(71) Applicant: Olympus Corporation, Hachioji-shi Tokyo 192-8507 (JP)
(72) Inventor: KAWANO, Yoshihiro, Tokyo 192-8507 (JP)
(74) Representative: Gunzelmann, Rainer
(86) International application number: PCT/JP2016/065965
(87) International publication number: WO 2017/018047

(57) **Abstract**

A desired lesion is observed without preparing a large number of specimens. Provided is a specimen staining system (1) including: a staining device (2) that can selectively perform multiple types of staining on a specimen on a glass slide (X); an observation device (3) that can obtain an image of the stained specimen stained by the staining device (2); a transfer device (4) that transfers the glass slide (X) loaded with the specimen between the observation device (3) and the staining device (2); and a controller (5) that controls the transfer device (4) and the staining device (2) so as to select the type of staining based on the image of the specimen obtained by the observation device (3) and perform the staining.

## Description

### {Technical Field}

The present invention relates to specimen staining systems.

### {Background Art}

In the related art, there are known methods for staining biological samples (for example, see Patent Literature 1).

The staining method in Patent Literature 1 is used to correct the contact of a staining agent and a decolorizing agent based on the difference image of images obtained before and after contact of the staining agent and the decolorizing agent with the sample.

### {Citation List}

### {Patent Literature}

{PTL 1} Japanese Unexamined Patent Application Publication No. 2014-134544

### {Summary of Invention}

### {Technical Problem}

However, because the method for staining a biological sample varies depending on the type of lesion to be observed, the staining method in Patent Literature 1, in which the contact of the staining agent and the decolorizing agent with one sample is corrected, is inconvenient because a large number of specimens need to be prepared according to the number of types of lesions to be observed.

The present invention has been made in view of the above-described circumstances, and an object thereof is to provide a specimen staining system with which a desired lesion can be observed without preparing a large number of specimens.

### {Solution to Problem}

To achieve the above-described object, the present invention provides the following solutions.

An aspect of the present invention is a specimen staining system including: a staining device that can selectively perform multiple types of staining on a specimen on a glass slide; an observation device that can obtain an image of the specimen stained by the staining device; a transfer device that transfers the glass slide loaded with the specimen between the observation device and the staining device; and a controller that controls the transfer device and the staining device so as to select the type of staining based on the image of the specimen obtained by the observation device and perform the staining.

According to this aspect, the specimen loaded on the glass slide is attached to the staining device, the selected type of staining is performed thereon, the transfer device is actuated to transfer the glass slide loaded with the stained specimen to the observation device, and an image of the stained specimen loaded on the transferred glass slide is measured in the observation device. Then, based on the obtained image, the controller selects the type of staining to be subsequently performed on the specimen and actuates the transfer device to transfer the glass slide from the observation device to the staining device.

In the staining device to which the glass slide loaded with the specimen has been transferred, staining of the type selected by the controller is performed, and the above-described process is repeated. As a result, multiple types of staining suitable for observation of different types of specimens are sequentially performed, and a desired observation result can be obtained. In this case, there is no need to load the same specimen on a large number of glass slides, and it is possible to reach a desired observation result without wasting specimens.

In the above-described aspect, the controller may include a determination unit that determines, based on the image of the specimen, the result of staining performed on the specimen, and a storage unit that stores a decision tree specifying the order of determination by the determination unit and the type of staining to be subsequently performed according to the determination result.

In this configuration, when the observation device obtains an image of the stained specimen, the image is forwarded to the determination unit of the controller, and the result of staining is determined therein. The controller, based on the result of determination by the determination unit, selects the type of staining to be subsequently performed, specified in the decision tree stored in the storage unit, and controls the transfer device and the observation device. As a result, it is possible to sequentially perform multiple types of staining on the same specimen and to efficiently obtain a desired observation result.

In the above-described aspect, the determination unit may determine the result of staining based on the area of a region in the stained specimen in which the staining intensity is higher than a predetermined threshold.

In a specimen that has been subjected to a predetermined type of staining, when the staining intensity is higher than a predetermined threshold, it can be determined that a target substance is present. Hence, the determination unit can easily determine the result of staining based on the area of the region in which the staining intensity is higher than the predetermined threshold.

Herein, "based on the area of the region" includes, in addition to the case where it means "based on the area value itself of the region in the image obtained by the observation device", the case where it means "based on the area ratio of the region with respect to the overall image area".

### {Advantageous Effects of Invention}

The present invention provides an advantage in that it is possible to observe a desired lesion without preparing a large number of specimens.

### {Brief Description of Drawings}

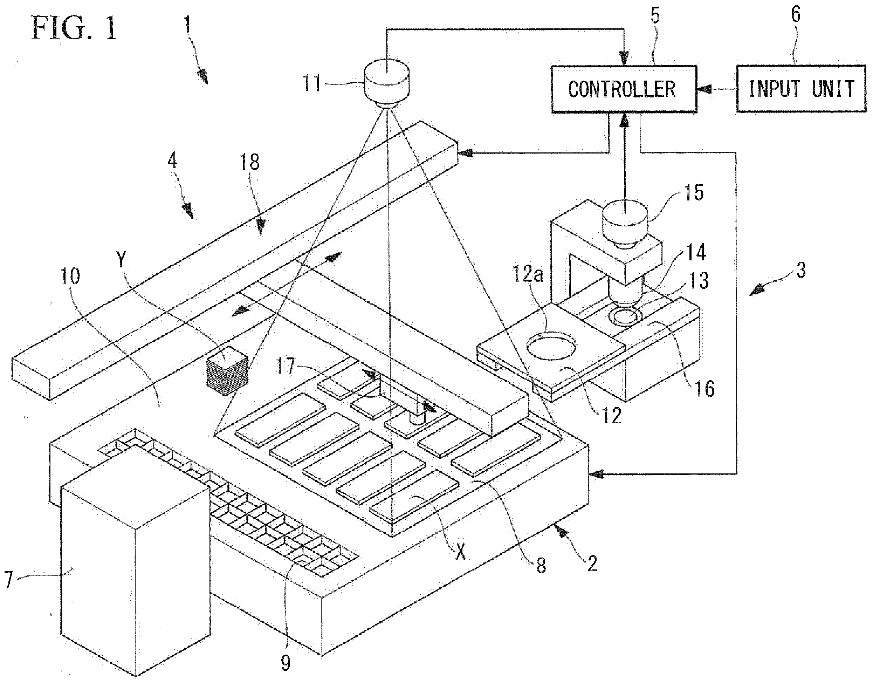

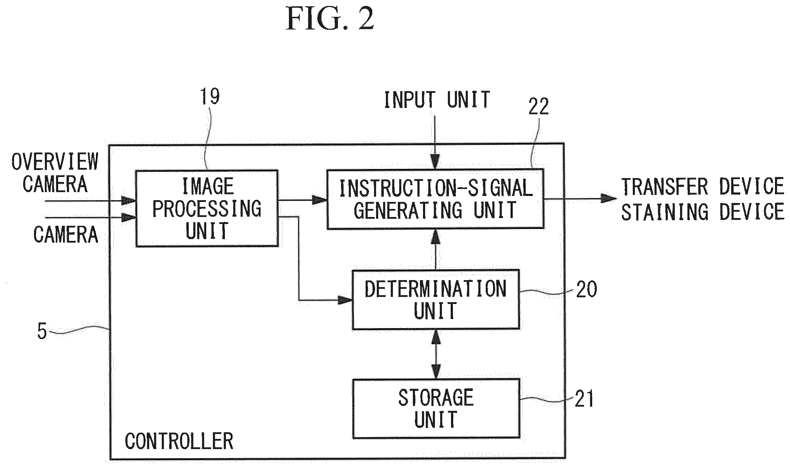

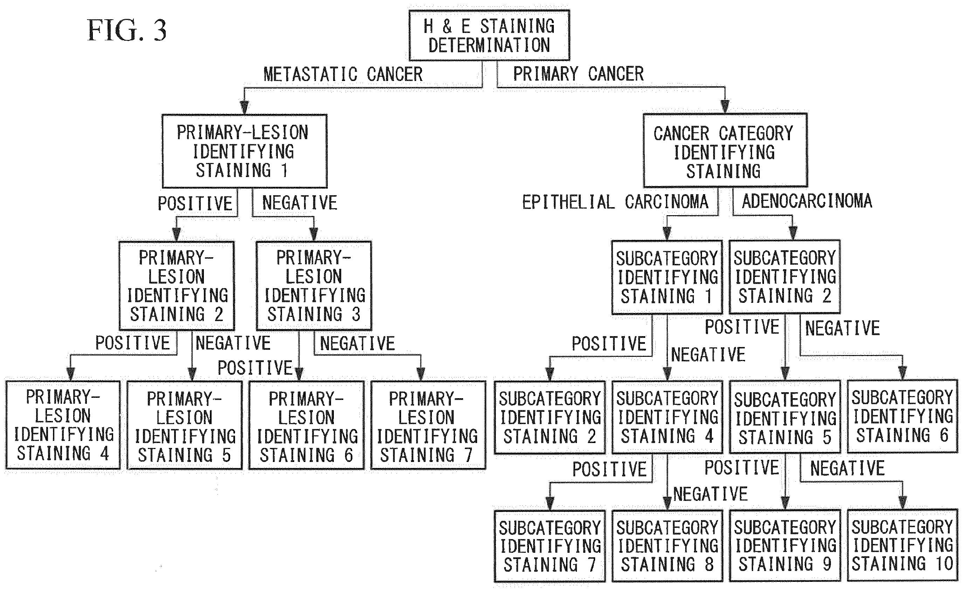

{Fig. 1} Fig. 1 shows the overall configuration of a specimen staining system according to an embodiment of the present invention.
{Fig. 2} Fig. 2 is a block diagram showing a controller of the specimen staining system in Fig. 1.
{Fig. 3} Fig. 3 shows an example decision tree stored in a storage unit provided in the controller of the specimen staining system in Fig. 1.

### {Description of Embodiments}

A specimen staining system 1 according to an embodiment of the present invention will be described below with reference to the drawings.

As shown in Fig. 1, the specimen staining system 1 according to this embodiment includes: a staining device 2 that carries, in a positioned state, one or more glass slides X loaded with a specimen and stains the specimen on the glass slide X; a microscope (observation device) 3 for observing the stained specimen on the glass slide X; a transfer device 4 that transfers the glass slide X between the microscope 3 and the staining device 2; a controller 5 that controls the transfer device 4 and the staining device 2 based on the result of observation by the microscope 3; an input unit 6 via which identification information of the specimen is input; and a slide storage 7 that stores the glass slide X after observation.

The staining device 2 has a staining area 8 in which one or more glass slides X are carried in a positioned state and in which staining is performed; multiple reagent containers 9 for separately storing multiple types of reagents; a cover-glass placement space 10 in which multiple cover glassed Y are disposed in a stacked state; and an overview camera 11 having a field of view in which at least the staining area 8 is located.

The staining area 8 is provided with a heater (not shown) for heating the area in which the glass slides X are carried in a positioned state. Furthermore, the staining area 8 has drainage grooves (not shown) for draining the reagent, cleaning liquid, or the like supplied to the specimen on one glass slide X for staining or cleaning so that it does not touch other glass slides X.

The microscope 3 includes: a stage 12 for carrying a glass slide X loaded with a stained specimen; an illuminating part 13 disposed below the stage 12 to irradiate the specimen with illumination light; an objective lens 14 disposed vertically above the specimen loaded on the stage 12 at a short distance therefrom to collect light transmitted through the specimen; and a camera 15 that captures an image of the light collected by the objective lens 14. The stage 12 has a through-hole 12a for allowing the illumination light to pass therethrough and is supported by a rail 16 so as to reciprocate between a transfer position, at which the glass slide X is transferred to and from the staining device 2, and an observation position, at which the glass slide X is disposed between the illuminating part 13 and the objective lens 14.

In this configuration, by causing the glass slide X received on the stage 12 located at the transfer position to slide along the rail 16, the microscope 3 can position the glass slide X at the observation position vertically below the objective lens 14 and can obtain a highly magnified image of the stained specimen. After the observation is finished, it is possible to move the stage 12 again to the transfer position to give the glass slide X to the transfer device 4.

The transfer device 4 includes, for example, a suction head 17 that sucks the glass slide X and a two-axis translation mechanism 18 that can horizontally move the suction head 17 in two directions. The transfer device 4 has such an operation range that the suction head 17 can be moved in the range including the staining area 8, reagent containers 9, and cover-glass placement space 10 of the staining device 2, the stage 12 of the microscope 3, and the slide storage 7. The suction head 17 includes, in addition to a suction part for sucking a glass slide X and a cover glass sheet Y, a suction nozzle that can suck the reagent or the like in the reagent containers 9.

The controller 5 is formed of a computer and, as shown in Fig. 2, includes: an image processing unit 19 that processes image signals input from the overview camera 11 and the camera 15 of the microscope 3; a determination unit 20 that determines the result of staining based on the image obtained by the camera 15 of the microscope 3; a storage unit 21 that stores a predetermined decision tree; and an instruction-signal generating unit 22 that generates instruction signals to the transfer device 4 and the staining device 2 based on the specimen-identification information input from the input unit 6, the image obtained by the overview camera 11, or the result of determination by the determination unit 20.

More specifically, the controller 5 identifies the coordinates of the multiple glass slides X disposed in the staining area 8 by processing the image signal obtained by the overview camera 11 and generates an instruction signal to the transfer device 4 to move the suction head 17 to the position of the glass slide X corresponding to the specimen-identification information input from the input unit 6.

Furthermore, the determination unit 20 of the controller 5 refers to the decision tree in the storage unit 21 based on the result of observation by the microscope 3 and selects the type of staining to be performed subsequently, according to the observation result.

Fig. 3 shows an example decision tree.

This decision tree is used to diagnose a cancer in a certain organ; first, in an image of a specimen of a tissue stained by H and E, whether the cancer is a metastatic cancer or a primary cancer is determined from the shape of the stained tissue.

Next, when the cancer is determined to be a metastatic cancer, staining for identifying the primary lesion is performed. Then, an image of the stained specimen is obtained with the microscope 3, and whether the presence of the target substance is positive or negative is determined based on the area of a region in which the staining intensity in the image is higher than a predetermined threshold. When the determination result is positive, staining for more precisely identifying the primary lesion is performed, and whether the presence of the target substance is positive or negative is determined based on the obtained image. When the determination result is negative, the target substance is changed, and staining for identifying another primary lesion is performed.

Meanwhile, when the cancer is determined to be a primary cancer, staining for determining whether the cancer is an epithelial carcinoma or an adenocarcinoma is performed. Then, an image of the stained specimen is obtained with the microscope 3, and whether the cancer is an epithelial carcinoma or an adenocarcinoma is determined based on the area of the region in which the staining intensity in the image is higher than the predetermined threshold.

Whether the cancer is determined to be an epithelial carcinoma or is determined to be an adenocarcinoma, in either case, performing another type of staining on the specimen and observing the specimen for determining the subtype of the cancer are repeated several times to determine the subcategory of the cancer.

The controller 5 controls, in each sequence of the decision tree, the transfer device 4 and the staining device 2 so as to make them perform a certain type of staining specified in that sequence. Specifically, the controller 5 controls the transfer device 4 to make it transfer a glass slide X from the stage 12 of the microscope 3 to the staining area 8 of the staining device 2 and controls the staining device 2 to make it stain the specimen on the glass slide X with a reagent to be used to perform the type of staining specified in the sequence. After the staining is completed, the transfer device 4 is controlled again to transfer the glass slide X from the staining device 2 to the microscope 3, and a new image is obtained.

Once all the sequences in any of the routes specified in the decision tree have been performed, the controller 5 controls the transfer device 4 to make it transfer the glass slide X to the staining device 2, and, in the staining device 2, to make the suction head 17 suck one cover glass sheet Y in the cover-glass placement space 10 and bring it into contact with the specimen on the glass slide X. Furthermore, the controller 5 controls the transfer device 4 to make it store the glass slide X, with which the cover glass sheet Y is in contact, in the slide storage 7.

The operation of the thus-configured specimen staining system 1 according to this embodiment will be described below.

When a specimen is observed by using the specimen staining system 1 according to this embodiment, the specimen is loaded on a glass slide X, and, without a cover glass sheet Y thereon, the glass slide X is positioned at a predetermined position in the staining area 8 of the staining device 2. According to the first sequence in the decision tree stored in the storage unit 21, the controller 5 instructs the staining device 2 and the transfer device 4 to perform H & E staining on the specimen on the glass slide X. The transfer device 4, according to the instruction from the controller 5, moves the suction head 17 and makes the suction head 17 suck Hematoxylin and Eosin staining liquids from the reagent container 9 and supply them to the specimen.

The staining device 2, according to the instruction from the controller 5, heats the specimen with a heater for a period of time needed for H & E staining. Upon completion of the staining, the transfer device 4 sucks the glass slide X loaded with the stained specimen, transports it, and transfers it to the stage 12 of the microscope 3. In the microscope 3, the specimen on the glass slide X loaded on the stage 12 is irradiated with the illumination light, the light transmitted through the specimen is collected by the objective lens 14, and an image thereof is captured by the camera 15.

The controller 5 determines, by pattern recognition, whether the cancer is a metastatic cancer or a primary cancer, based on the image obtained by the camera 15. Whether the cancer is a metastatic cancer or a primary cancer, in either case, staining of the same specimen with another type of reagent and observation are repeated according to the subsequent sequence of the decision tree. Once all the sequences in any of the routes in the decision tree have been completed, the glass slide X is moved to the staining device 2, a cover glass sheet Y is brought into contact therewith, and the glass slide X is stored in the slide storage 7.

The thus-configured specimen staining system 1 according to this embodiment has an advantage in that there is no need to prepare a large number of specimens because multiple types of staining are performed on a single specimen.

Although pathologists diagnose by viewing the forms of tissues stained by H and E, because it is difficult to perform all diagnosis by the form alone, additional immunodiagnosis needs to be performed. When a more reliable diagnosis result is to be obtained by pathological diagnosis, when the cancer is a primary cancer, it is important to determine the type of the cancer (e.g., whether the cancer is an epithelial carcinoma or an adenocarcinoma).

To determine the type of the cancer, it is necessary to determine the subcategory of the cancer. When a molecular target drug is used, it is necessary to determine whether the drug works or not by pathological diagnosis.

When a perfect result is wanted, a large number of immunologic tests need to be simultaneously performed, requiring immunostaining on specimens on 30 to 50 glass slides X, depending on the type of the cancer.

In this aspect, when the organ is determined, preset types of staining for detecting the cancer that statistically frequently occurs in that organ and the cancer for which there are drugs are sequentially performed on a single specimen. Hence, it is possible to obtain a desired result without preparing a large number of specimens.

In this embodiment, whether the presence of the target substance is positive or negative is determined based on the area of the region (high-intensity region) in which the staining intensity in the image is higher than the predetermined threshold. However, the determination may be performed based on the ratio of the area of the high-intensity region to the area of the overall image. Alternatively, the determination may be performed by averaging the areas or the area ratios at multiple portions in the specimen.

### {Reference Signs List}

- 1: specimen staining system
- 2: staining device
- 3: microscope (observation device)
- 4: transfer device
- 5: controller
- 20: determination unit
- 21: storage unit
- X: glass slide

## Claims

1. A specimen staining system comprising:
a staining device that can selectively perform multiple types of staining on a specimen on a glass slide;
an observation device that can obtain an image of the specimen stained by the staining device;
a transfer device that transfers the glass slide loaded with the specimen between the observation device and the staining device; and
a controller that controls the transfer device and the staining device so as to select the type of staining based on the image of the specimen obtained by the observation device and perform the staining.

2. The specimen staining system according to Claim 1,
wherein the controller includes a determination unit that determines, based on the image of the specimen, the result of staining performed on the specimen, and a storage unit that stores a decision tree specifying the order of determination by the determination unit and the type of staining to be subsequently performed according to the determination result.

3. The specimen staining system according to Claim 2,
wherein the determination unit determines the result of staining based on the area of a region in the stained specimen in which the staining intensity is higher than a predetermined threshold.
